# EUROPÄISCHE PATENTANMELDUNG

(11) **EP 1 295 576 A2**
(43) Veröffentlichungstag der Anmeldung: **26.03.2003**
(21) Anmeldenummer: 02020744.5
(22) Anmeldetag: 16.09.2002
(51) Int. Cl.: A61F 2/36

(54) **Hüftgelenkprothese mit anschlaggeschütztem Prothesenschaft**

(30) Priorität: 19.09.2001 DE 10146075; 27.04.2002 DE 10218978
(71) Anmelder: CeramTec AG Innovative Ceramic Engineering, 73207 Plochingen (DE)
(72) Erfinder: Bunz, Uwe, Dipl.-Ing., 72649 Wolfschlugen (DE)
(74) Vertreter: Uppena, Franz, Dr.

(57) **Zusammenfassung**

Die Erfindung betrifft eine Hüftgelenkprothese mit einer äußeren Metallschale (5), einem inneren Pfanneneinsatz (4) in dem ein Kugelkopf (3) artikuliert, der Kugelkopf (3) mit einem metallischen Prothesenschaft (2) der femoralen Schaftkomponente verbunden ist und die Bewegung des Prothesenschaftes (2) durch einen Anschlag an den Rand des Pfanneneinsatzes (4) begrenzt ist.

Zur Verbesserung der Hüftgelenkprothese in Bezug auf den Anschlag des Prothesenschaftes an den Pfanneneinsatz wird vorgeschlagen, dass im Bereich des Anschlages des Prothesenschaftes (2) an den Pfanneneinsatz (4) auf der Umfangsfläche des Prothesenschaftes (2) ein weiches biokompatibles Dämpfungselement (1) aufgebracht ist.

## Beschreibung

Die Erfindung betrifft eine Hüftgelenkprothese mit einer äußeren Metallschale, einem inneren Pfanneneinsatz in dem ein Kugelkopf artikuliert, der Kugelkopf mit einem metallischen Prothesenschaft der femoralen Schaftkomponente verbunden ist und die Bewegung des Prothesenschaftes durch einen Anschlag an den Rand des Pfanneneinsatzes begrenzt ist.

Bei heutigen Prothesen wird die Bewegung der femoralen Schaftkomponente durch Maßnahmen an der Hüftgelenkspfanne limitiert. Es gibt verschiedene Ausführungsformen, z. B.
- Einen erhöhten Metallrand
- Einen erhöhten PE-Rand (PE = Polyethylen) bei Sandwich-Systemen (z. B. US 5,879,404)
- den PE-, Keramik- oder Metalleinsatz selbst
Hierbei wird der Prothesenschaft zumeist nicht auf den Anschlagpartner hin optimiert. Bei Schaftprothesen sind oft drei Positionen des Prothesenschaftes zur Hüftgelenkspfanne möglich.

Aus diesen genannten konstruktiven Ausführungen resultieren verschiedene Nachteile:
- Eine Optimierung der Schaftkomponente hinsichtlich Anschlag kann nicht erfolgen. Dadurch ist eine Adaptierung der Anschlaggeometrie zur Minimierung der vorliegenden Flächenpressung nicht möglich. Ferner ist eine Anpassung der Schaftkomponente auf alle mögliche Einbaulagen ebenfalls nicht möglich
- Bei Anschlag des metallischen Schaftes an einen Metall- oder Keramikeinsatz wird Metallabrieb freigesetzt, der zu den bekannt negativen Auswirkungen führen kann.
- Bei Anschlag an PE kann aufgrund der hohen Flächenpressungen ein Fließen des PE nicht vermieden werden. Das Implantat wird instabil und es kann bei starker PE-Deformation zu Situationen kommen, in denen die Funktionstüchtigkeit des Implantats nicht gewährleistet ist.
- Das PE in der Hüftgelenkspfanne ist nicht auf möglichst gute Dämpfung des Schaftanschlages optimiert, da Material und Gestalt für den Einsatz als Gleitpartner optimiert sind. Es ist vor allem keine definierte Dämpfung des Anschlags möglich. Bei erhöhten Impulsenergien kann dies das Versagen eines Implantates zur Folge haben.

Der Erfindung liegt die Aufgabe zu Grunde, eine Hüftgelenkprothese nach dem Oberbegriff des Anspruchs 1 in Bezug auf den Anschlag des Prothesenschaftes an den Pfanneneinsatz zu verbessern.

Erfindungsgemäß wird diese Aufgabe dadurch gelöst, dass im Bereich des Anschlages des Prothesenschaftes an den Pfanneneinsatz auf der Umfangsfläche des Prothesenschaftes ein weiches, biokompatibles Dämpfungselement aufgebracht ist.

In bevorzugter Ausgestaltung ist das Dämpfungselement aus einem Kunststoff hergestellt, z. B. PE (Polyethylen), PEI (Polyetherimid), Silikon oder TPE (thermoplastischer Elastomer), um nur einige zu nennen.

Vorteilhafterweise ist die geometrische Ausgestaltung des Dämpfungselements an den korrespondierenden Rand des Pfanneneinsatzes angepasst, d. h. der Rand des Pfanneneinsatzes kann in das Dämpfungselement beim Anschlag eingreifen oder umgekehrt.

Hierzu sind bevorzugt in der zum Pfanneneinsatz gewandten Fläche des Dämpfungselements Einbuchtungen für den korrespondierenden Rand des Pfanneneinsatzes angeordnet. Diese Einbuchtungen bilden den Abdruck des Randes des Pfanneneinsatzes.

Es kann von Vorteil sein, wenn das Dämpfungselement in seiner axialen Ausdehnung bis in den Kugelkopf hineinreicht. Hierdurch kann eine extrem gute Fixierung erreicht werden.

In bevorzugter Ausgestaltung ist das Dämpfungselement ein Ring, vorteilhafterweise ein Ring mit einem Schlitz, der z. B. axial, schräg und/oder sägezahnartig ausgeführt sein kann. Durch den Schlitz ist eine einfache Montage des Dämpfungselements auf dem Prothesenschaft möglich.

Die Enden des Schlitzes sind vorteilhafterweise sich überlappend angeordnet, so dass im Falle eines Anschlages immer eine Dämpfung erzielt wird.

In bevorzugter Ausführungsform sind zwischen dem Dämpfungselement und dem Prothesenschaft Hohlräume zur Aufnahme von Körperflüssigkeiten angeordnet. Beim Anschlag des Prothesenschaftes an den Pfanneneinsatz werden diese Hohlräume gestaucht bzw. zusammengedrückt und die Körperflüssigkeit herausgedruckt, so dass sich eine Dämpfung ergibt.

Die Idee der vorliegenden Erfindung ist es eine im Vergleich zum metallischen Schaft weiche biokompatible Schicht auf den Prothesenschaft aufzubringen. Die geometrische Ausgestaltung dieser Schicht wird an die korrespondierende Hüftgelenkspfanne angepasst um die auftretenden Flächenpressungen im Fall eines Anschlages zu minimieren. Die konstruktive Ausführung der Hüftgelenkspfanne kann eine direktgeklemmte Ausführung, eine Sandwich-Ausführung, ein Pressfitoder Schraubpfannen-System umfassen. Das Material dieser Zwischenschicht ist vorzugsweise ein Kunststoff (PE, PEI, Silikon, TPE,...).

Nachfolgend die Vorteile dieser Erfindung im Vergleich zum Stand der Technik:
- Eine Optimierung hinsichtlich eines vergrößerten Bewegungsspielraumes (ROM) ist durch die Systembetrachtung möglich. Eine Anschlagoptimierung wurde bislang entweder am Prothesenschaft oder der Hüftgelenkspfanne vorgenommen. Diese definierten Anschläge wurden zum Teil (erhöhter Metallrand) mit einer Einschränkung des ROM erkauft. Durch die Betrachtung des Gesamtsystems und Abstimmung hinsichtlich Anschlag können in der weichen biokompatiblen Schicht Aussparungen bzw. Einbuchtungen eingebracht werden, die den ROM vergrößern.
- Eine Optimierung des Betriebszustandes Anschlag ist möglich:
   Dazu gehört eine Adaptierung der Anschlaggeometrie der weichen biokompatiblen Schicht zur Minimierung der vorliegenden Flächenpressung und eine Anpassung der Schaftkomponente auf alle möglichen Einbaulagen. Diese Einbaulagen werden definiert durch unterschiedliche Halslängen der Kugelköpfe.
- Bei Anschlag des Schaftes kann durch geeignete Materialwahl der weichen biokompatiblen Schicht nur wenig biokompatibles Material freigesetzt werden (kein Metallabrieb / kein PE-Fließen)
- Eine definierte Dämpfung des Anschlags ist möglich:
   Durch die Verwendung von Materialien mit hoher innerer Dämpfung und durch die Funktionstrennung von Gleitpartner und Dämpfungselement sind sowohl eine innere Dämpfung als auch konstruktive Dämpfungselemente möglich. Die Dämpfung wird über den Wert der inneren Dämpfung hinaus durch definiertes Entweichen von Körperflüssigkeiten (im wesentlichen Wasser) erreicht.

Weitere Merkmale der Erfindung ergeben sich aus den Figuren, die nachfolgend beschrieben sind.

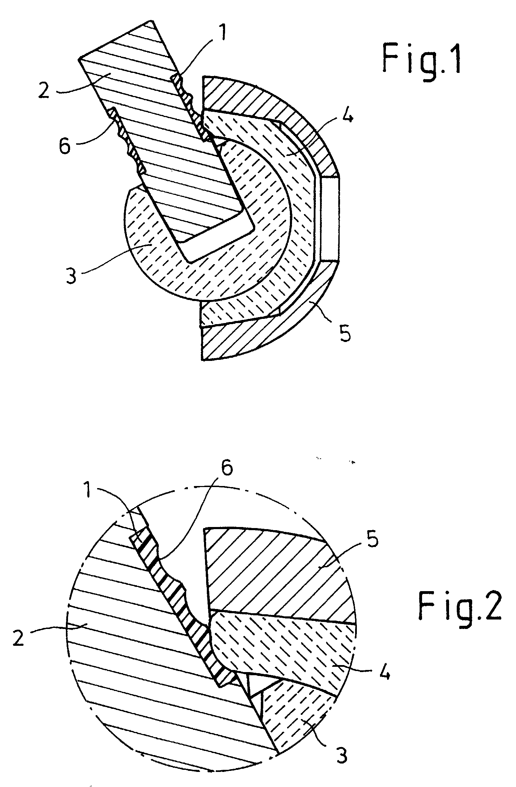

Fig. 1 zeigt in einem Querschnitt eine erfindungsgemäße Ausgestaltung einer Hüftgelenkprothese. Fig. 2 zeigt dieselbe Ausführungsform in einem vergrößerten Ausschnitt.

In einer äußeren Metallschale 5 ist ein keramischer Pfanneneinsatz 4 eingesetzt. Am unteren Ende ist in der Metallschale 5 eine Öffnung angeordnet.

Im Pfanneneinsatz 4 artikuliert ein keramischer Kugelkopf 3, der mit einem metallischen Prothesenschaft 2 der femoralen Schaftkomponente verbunden ist. Hierzu ist der Prothesenschaft 2 meist mit einer konischen Klemmverbindung in einer konischen Bohrung im Kugelkopf 3 verankert.

Die Bewegung des Prothesenschaftes 2 ist durch einen Anschlag an den Rand des Pfanneneinsatzes 4 begrenzt. Im Bereich des Anschlages ist auf der Umfangsfläche des Prothesenschaftes 2 ein weiches biokompatibles Dämpfungselement 1 angeordnet. In der hier gezeigten Ausführungsform ist dieses Dämpfungselement als ein auf den Prothesenschaft 2 aufgeschobenen Ring ausgebildet. Der Ring besteht aus einem Kunststoff, wie z. B. Polyethylen (PE), PEI, Silikon oder TPE.

Im Prothesenschaft 2 ist ein Einschnitt vorgesehen, in dem der Ring bzw. das Dämpfungselement 1 eingesetzt ist.

Die geometrische Ausgestaltung des Dämpfungselements 1 ist an den korrespondierenden Rand des Pfanneneinsatzes 4 angepasst und zwar durch Einbuchtungen 6. Diese Einbuchtungen 6 sind so geformt, dass der Rand des Pfanneneinsatzes 4 in diese eingreifen kann. Hierdurch ist die maximale Bewegungsfreiheit des Prothesenschaftes 2 vergrößert.

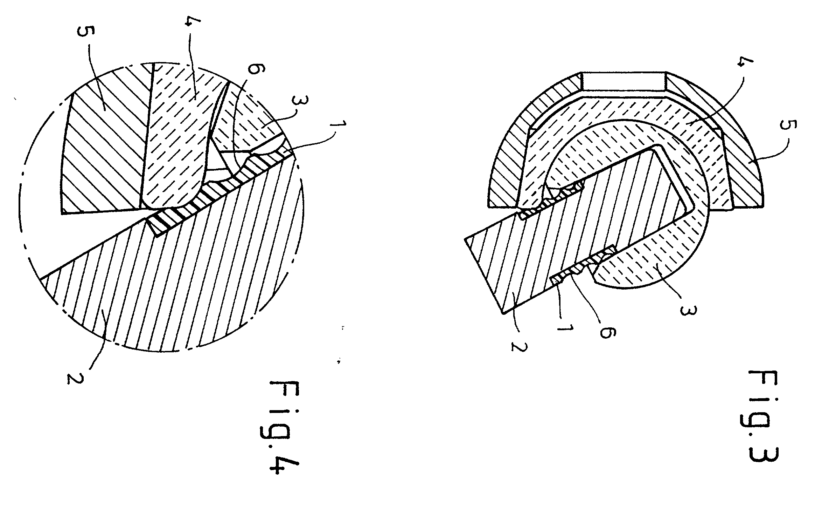

Die Figuren 3 und 4 zeigen eine ähnliche Ausführungsform wie die in den Figuren 1 und 2, nur ist hier das Dämpfungselement 1 in seiner axialen Ausdehnung bis in den Kugelkopf 3, bzw. die Bohrung im Kugelkopf 3, hineinreichend ausgebildet.

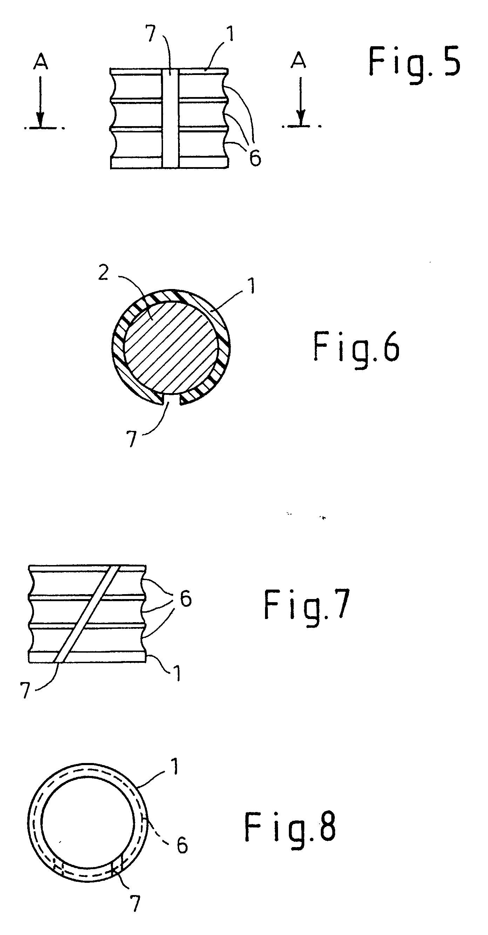

Fig. 5 zeigt als Ansicht ein Dämpfungselement 1 mit einem axialen Schlitz 7, der zum Montieren des Dämpfungselements 1 auf dem Prothesenschaft 2 dient. Das Dämpfungselement 1 ist als Ring geformt und hat auf seiner Außenfläche drei umlaufende Einbuchtungen 6. Fig. 6 zeigt einen Schnitt entlang der Linie A-A von Fig. 5 durch das den Prothesenschaft 2 umgebende Dämpfungselement 1.

Fig. 7 zeigt als Ansicht ein Dämpfungselement 1 ähnlich wie das in Fig. 5 gezeigte Dämpfungselement, jedoch ist hier der Schlitz 7 schräg verlaufend angeordnet. Umlaufende Einbuchtungen 6 sind ebenfalls vorhanden. Fig. 8 zeigt eine Aufsicht auf das Dämpfungselement nach Fig. 7.

Fig. 9 zeigt als Ansicht ein Dämpfungselement 1 mit einem Schlitz 7, der sägezahnartig ausgeführt ist. In allen Ausführungsvarianten können sich die Enden des Schlitzes 7 überlappen. Die Einbuchtungen 6 sind dieselben wie in den vorhergehenden Figuren.

Die Figuren 10a und 10b zeigen ein Dämpfungselement 1 mit Hohlräumen 8 auf der Innenseite zur Aufnahme von Körperflüssigkeiten. Diese Hohlräume 8 sind wie die Einbuchtungen 6 wellenförmig ausgebildet und sind durch einen Kanal 9 miteinander verbunden.

Fig. 10b zeigt einen Schnitt entlang der Linie A-A in Fig. 10a.

Bei einem Anschlag des Prothesenschaftes 2 an den Rand des Pfanneneinsatzes werden diese Hohlräume 8 zusammengedrückt und die darin enthaltende Körperflüssigkeit - im wesentlichen Wasser, H₂O ― herausgedrückt. Hierdurch ergibt sich eine dämpfende Eigenschaft der Hohlräume 8.

Die Fig. 11 zeigt einen Schnitt durch einen Prothesenschaft 2 mit aufgesetztem Kugelkopf 3 und aufgesetztem Dämpfungselement 1 mit inneren Hohlräumen 8.

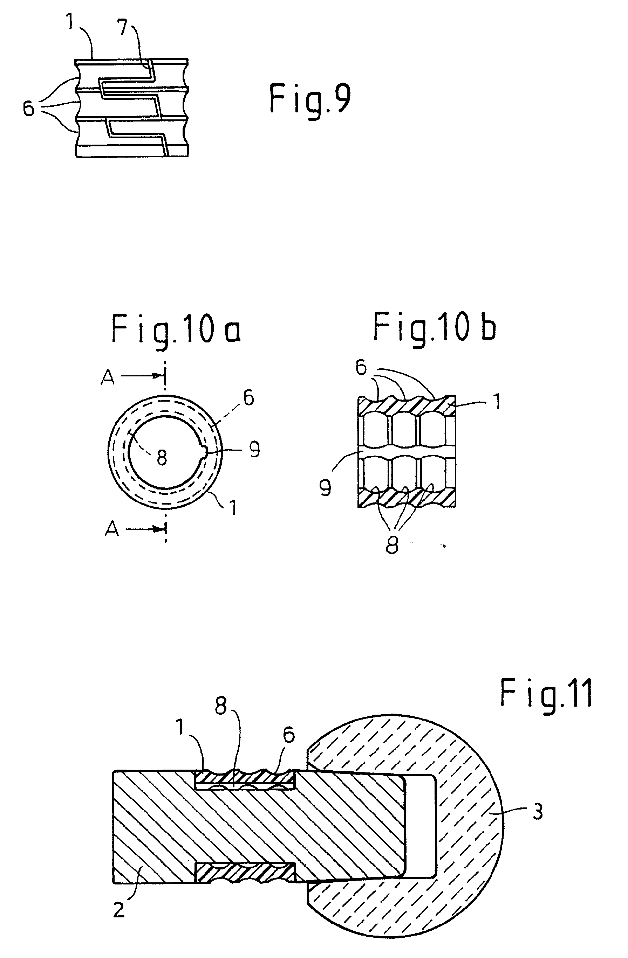

Auf der oberen Ansicht von Fig. 11 sind auf dem Prothesenschaft 2 Erhebungen angeordnet und das Dämpfungselement 1 ist flach ausgebildet, wodurch die Hohlräume 8 geschaffen sind.

Auf der unteren Ansicht von Fig. 11 ist eine Ausführungsform gemäß Fig. 10 a, b abgebildet, d. h. die Hohlräume 8 sind in das Dämpfungselement 1 integriert durch wellenförmige Einbuchtungen.

## Patentansprüche

1. Hüftgelenkprothese mit einer äußeren Metallschale (5), einem inneren Pfanneneinsatz (4) in dem ein Kugelkopf (3) artikuliert, der Kugelkopf (3) mit einem metallischen Prothesenschaft (2) der femoralen Schaftkomponente verbunden ist und die Bewegung des Prothesenschaftes (2) durch einen Anschlag an den Rand des Pfanneneinsatzes (4) begrenzt ist, **dadurch gekennzeichnet, dass** im Bereich des Anschlages des Prothesenschaftes (2) an den Pfanneneinsatz (4) auf der Umfangsfläche des Prothesenschaftes (2) ein weiches biokompatibles Dämpfungselement (1) aufgebracht ist.

2. Hüftgelenkprothese nach Anspruch 1, **dadurch gekennzeichnet, dass** das Dämpfungselement (1) ein Kunststoff ist.

3. Hüftgelenkprothese nach Anspruch 1 oder 2, **dadurch gekennzeichnet, dass** die geometrische Ausgestaltung des Dämpfungselements (1) an den korrespondierenden Rand des Pfanneneinsatzes (4) angepasst ist.

4. Hüftgelenkprothese nach Anspruch 3, **dadurch gekennzeichnet, dass** in der zum Pfanneneinsatz (4) gewandten Fläche des Dämpfungselementes (1) Einbuchtungen (6) für den korrespondierenden Rand des Pfanneneinsatzes (4) angeordnet sind.

5. Hüftgelenkprothese nach einem der Ansprüche 1 bis 4, **dadurch gekennzeichnet, dass** das Dämpfungselement (1) in seiner axialen Ausdehnung bis in den Kugelkopf (3) reicht.

6. Hüftgelenkprothese nach einem der Ansprüche 1 bis 5, **dadurch gekennzeichnet, dass** das Dämpfungselement (1) ein Ring ist.

7. Hüftgelenkprothese nach Anspruch 6, **dadurch gekennzeichnet, dass** im Ring ein Schlitz (7) angeordnet ist.

8. Hüftgelenkprothese nach Anspruch 7, **dadurch gekennzeichnet, dass** der Schlitz (7) axial, schräg und/oder sägezahnartig ausgeführt ist.

9. Hüftgelenkprothese nach Anspruch 7 oder 8, **dadurch gekennzeichnet, dass** die Enden des Schlitzes (7) sich überlappend angeordnet sind.

10. Hüftgelenkprothese nach einem der Ansprüche 1 bis 9, **dadurch gekennzeichnet, dass** zwischen dem Dämpfungselement (1) und dem Prothesenschaft (2) Hohlräume (8) zur Aufnahme von Körperflüssigkeiten angeordnet sind.
